# EUROPEAN PATENT SPECIFICATION

(11) **EP 4 295 781 B1**
(45) Date of publication and mention of the grant of the patent: **11.06.2025**
(21) Application number: 23180926.0
(22) Date of filing: 22.06.2023
(51) Int. Cl.: A61B 8/00

(54) **IMAGE DISPLAY APPARATUS AND CONTROL METHOD OF IMAGE DISPLAY APPARATUS**
BILDANZEIGEVORRICHTUNG UND STEUERUNGSVERFAHREN FÜR BILDANZEIGEVORRICHTUNG
APPAREIL D'AFFICHAGE D'IMAGE ET PROCÉDÉ DE COMMANDE D'APPAREIL D'AFFICHAGE D'IMAGE

(30) Priority: 22.06.2022 JP 2022100084
(43) Date of publication of application: 27.12.2023
(73) Proprietor: FUJIFILM Corporation, Tokyo 106-8620 (JP)
(72) Inventor: KOSHINO, Riko, Kanagawa, 258-8538 (JP)
(74) Representative: Dehns Germany Partnerschaft mbB

(56) References cited:
- US-A1- 2021 030 392

## Description

### BACKGROUND OF THE INVENTION

### 1. Field of the Invention

The present invention relates to an image display apparatus and a control method of the image display apparatus which display a plurality of ultrasound images in which a lesion part of a subject is imaged.

### 2. Description of the Related Art

In the related art, in the medical field, an ultrasound diagnostic apparatus using an ultrasound image has been put to practical use. In general, an ultrasound diagnostic apparatus includes an ultrasound probe with a built-in transducer array, and an apparatus main body connected to the ultrasound probe, and the ultrasound diagnostic apparatus causes the ultrasound probe to transmit an ultrasound beam toward a subject, receives an ultrasound echo from the subject by the ultrasound probe, and electrically processes a reception signal thereof to generate an ultrasound image.

In a case where the ultrasonography is performed on the lesion part of the subject using the ultrasound diagnostic apparatus, a plurality of ultrasound images are usually acquired, but a wide variety of images may be included in the acquired ultrasound images. For example, ultrasound images of different image types such as a brightness (B) mode image, an image superimposed with Doppler information, and an image superimposed with elasticity information may be included. Even the same B-mode images may include images in which the positions of the lesion part are different, and images of which the sizes of the imaging range are different.

After the ultrasonography for the subject is ended, a radiologist checks and interprets the ultrasound image displayed on a viewer, and creates findings, and thus it is important to display the plurality of acquired ultrasound images in a proper order in order to improve the interpretation efficiency.

For example, JP2015-100661A discloses a medical image system that classifies a plurality of ultrasound images stored in a storage unit into a B-mode image represented in grayscale, and a color image, and then sorts the ultrasound images in time series to decide a display order of the plurality of ultrasound images. For example, US 2021/030392 A1 discloses an ultrasound system and a method for breast tissue imaging and annotation of breast ultrasound image.

### SUMMARY OF THE INVENTION

In the medical image system disclosed in JP2015-100661A, since the ultrasound images are classified into the grayscale image and the color image, it is possible to separately display the B-mode image, the image superimposed with Doppler information, and the image superimposed with elasticity information.

However, various images may be stored even for the B-mode images alone, and in the medical image system of JP2015-100661A, it is difficult to select images in which the same lesion part is imaged, from the plurality of stored ultrasound images, and to decide the display order according to the position and imaging range of the imaged lesion part. Therefore, there is a possibility that the interpretation efficiency may be lowered.

The present invention has been made in order to solve such a problem in the related art, and an object of the present invention is to provide an image display apparatus and a control method of the image display apparatus which can improve the interpretation efficiency of the plurality of ultrasound images.

According to the appended claims, the above object can be achieved.

According to the present invention, the probe positional information of the ultrasound probe, and the imaging feature information regarding the position of the lesion part and the imaging range in each of the plurality of ultrasound images are acquired, the ultrasound image conforming to the display layout selected by the user is extracted from the plurality of ultrasound images by referring to the probe positional information and the imaging feature information, and the extracted ultrasound image is displayed on the monitor according to the display layout. Therefore, it is possible to improve the interpretation efficiency of the plurality of ultrasound images.

### BRIEF DESCRIPTION OF THE DRAWINGS

Fig. 1 is a block diagram illustrating a configuration of an image display apparatus according to a first embodiment of the present invention.
Fig. 2 is a block diagram illustrating an internal configuration of a transmission and reception circuit in the first embodiment.
Fig. 3 is a block diagram illustrating an internal configuration of an image generation unit in the first embodiment.
Fig. 4 is a diagram illustrating an example of a body mark representing a breast.
Fig. 5 is a diagram illustrating an example of a body mark to which a probe mark is attached.
Fig. 6 is a diagram illustrating another example of a body mark to which a probe mark is attached.
Fig. 7 is a diagram illustrating still another example of a body mark to which a probe mark is attached.
Fig. 8 is a diagram illustrating an ultrasound image in which a lesion part is shown near the center of a screen.
Fig. 9 is a diagram illustrating an ultrasound image in which a lesion part is shown at a position shifted from the center of a screen in a horizontal direction.
Fig. 10 is a diagram illustrating an example of a display layout of an ultrasound image on a monitor.
Fig. 11 is a flowchart illustrating an operation of the first embodiment.
Fig. 12 is a diagram illustrating an example of an ultrasound image displayed on a monitor according to the first embodiment.
Fig. 13 is a block diagram illustrating a configuration of an image display apparatus according to a second embodiment.
Fig. 14 is a block diagram illustrating a configuration of an image display apparatus according to a third embodiment.
Fig. 15 is a flowchart illustrating an operation of the third embodiment.

### DESCRIPTION OF THE PREFERRED EMBODIMENTS

Hereinafter, embodiments of the invention will be described with reference to the accompanying drawings.

The description of configuration requirements described below is given on the basis of the representative embodiment of the present invention, but the present invention is not limited to such an embodiment.

In the present specification, a numerical range represented using "to" means a range including the numerical values before and after "to" as a lower limit value and an upper limit value.

In the present specification, the terms "same" and "identical" include an error range generally allowed in the technical field.

### First Embodiment

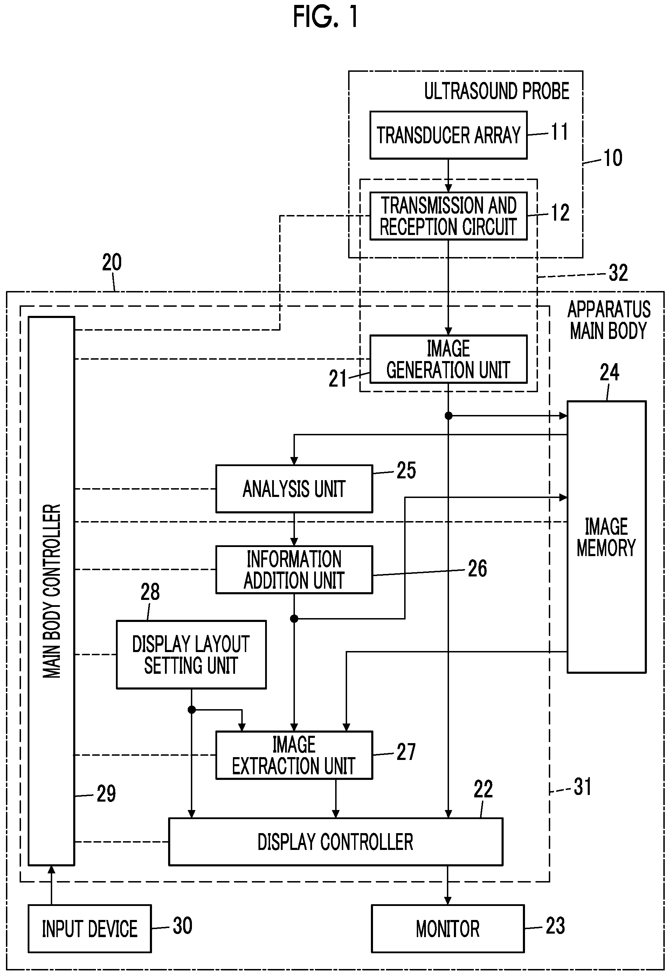

Fig. 1 illustrates a configuration of an image display apparatus according to a first embodiment of the present invention. The image display apparatus has a function as an ultrasound device that acquires an ultrasound image, and includes an ultrasound probe 10, and an apparatus main body 20. The ultrasound probe 10 and the apparatus main body 20 are connected to each other in a wired manner via a cable (not illustrated).

The ultrasound probe 10 includes a transducer array 11, and a transmission and reception circuit 12 is connected to the transducer array 11.

the apparatus main body 20 has an image generation unit 21 connected to the transmission and reception circuit 12 of the ultrasound probe 10, a display controller 22 and a monitor 23 are sequentially connected to the image generation unit 21, and an image memory 24 is connected to the image generation unit 21.

An analysis unit 25 is connected to the image memory 24, an information addition unit 26 and an image extraction unit 27 are sequentially connected to the analysis unit 25, and the display controller 22 is connected to the image extraction unit 27. The image memory 24 is connected to the information addition unit 26, and the image extraction unit 27 is connected to the image memory 24. Further, a display layout setting unit 28 is connected to the image extraction unit 27 and the display controller 22.

A main body controller 29 is connected to the image generation unit 21, the display controller 22, the image memory 24, the analysis unit 25, the information addition unit 26, the image extraction unit 27, and the display layout setting unit 28, and an input device 30 is connected to the main body controller 29. The transmission and reception circuit 12 of the ultrasound probe 10 is connected to the main body controller 29.

The image generation unit 21, the display controller 22, the analysis unit 25, the information addition unit 26, the image extraction unit 27, the display layout setting unit 28, and the main body controller 29 constitute a processor 31.

The transducer array 11 of the ultrasound probe 10 has a plurality of ultrasonic transducers arranged in a one-dimensional or two-dimensional manner. According to a drive signal supplied from the transmission and reception circuit 12, each of the transducers transmits an ultrasonic wave and receives a reflected wave from the subject to output an analog reception signal. For example, each transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

The transmission and reception circuit 12 causes the transducer array 11 to transmit the ultrasonic wave and generates a sound ray signal on the basis of a reception signal acquired by the transducer array 11, under the control of the main body controller 29.

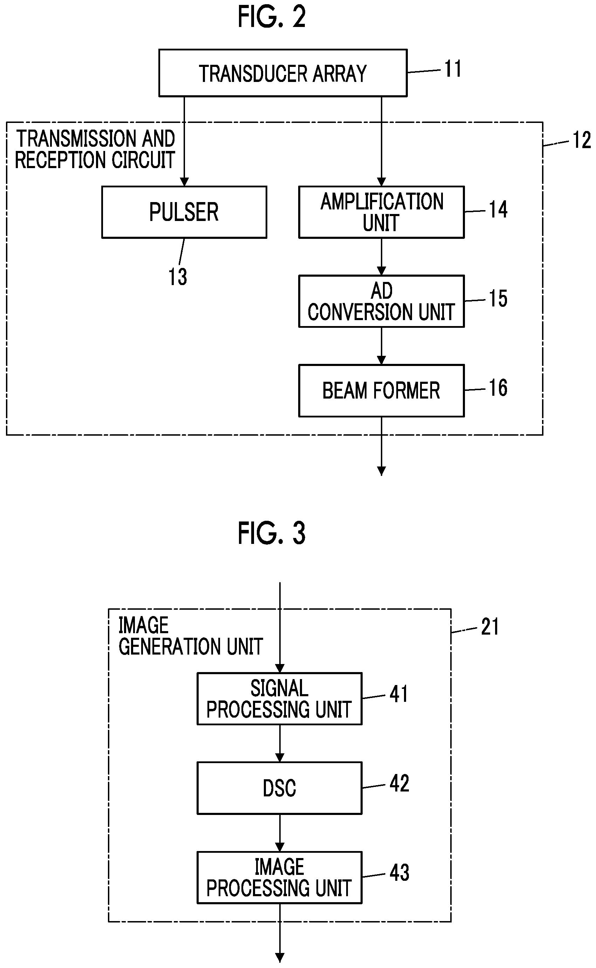

As illustrated in Fig. 2, the transmission and reception circuit 12 has a pulser 13 connected to the transducer array 11, and an amplification unit 14, an analog digital (AD) conversion unit 15, and a beam former 16 that are sequentially connected in series to the transducer array 11.

The pulser 13 includes, for example, a plurality of pulse generators, and the pulser 13 adjusts the amount of delay of each drive signal so that ultrasonic waves transmitted from the plurality of transducers of the transducer array 11 form an ultrasound beam on the basis of a transmission delay pattern selected according to the control signal from the main body controller 29, and supplies the obtained signals to the plurality of transducers. Thus, in a case where a pulsed or continuous-wave voltage is applied to the electrodes of the transducers of the transducer array 11, the piezoelectric body expands and contracts to generate pulsed or continuous-wave ultrasonic waves from each transducer. From the combined wave of these ultrasonic waves, an ultrasound beam is formed.

The transmitted ultrasound beam is reflected by a target, for example, a site of the subject, and the ultrasound echo propagates toward the transducer array 11 of the ultrasound probe 10. The ultrasound echo propagating toward the transducer array 11 in this manner is received by each transducer constituting the transducer array 11. In this case, each transducer constituting the transducer array 11 expands and contracts by receiving the propagating ultrasound echo to generate a reception signal that is an electric signal, and outputs the reception signal to the amplification unit 14.

The amplification unit 14 amplifies the signals input from each transducer constituting the transducer array 11, and transmits the amplified signals to the AD conversion unit 15. The AD conversion unit 15 converts the signal transmitted from the amplification unit 14 into digital reception data, and transmits the reception data to the beam former 16. The beam former 16 performs so-called reception focusing processing in which addition is performed by giving delays to respective pieces of the reception data converted by the AD conversion unit 15 according to a sound speed distribution or a sound speed set on the basis of a reception delay pattern selected according to the control signal from the main body controller 29. Through the reception focusing processing, a sound ray signal in which each piece of the reception data converted by the AD conversion unit 15 is phased and added and the focus of the ultrasound echo is narrowed is acquired.

As illustrated in Fig. 3, the image generation unit 21 of the apparatus main body 20 has a configuration in which a signal processing unit 41, a digital scan converter (DSC) 42, and an image processing unit 43 are sequentially connected in series.

The signal processing unit 41 generates an ultrasound image signal (B-mode image signal), which is tomographic image information regarding tissues inside the subject, by performing, on the sound ray signal sent from the transmission and reception circuit 12 of the ultrasound probe 10, correction of the attenuation due to the distance according to the depth of the reflection position of the ultrasonic wave and then performing envelope detection processing.

The DSC 42 converts (raster conversion) the ultrasound image signal generated by the signal processing unit 41 into an image signal according to a normal television signal scanning method.

The image processing unit 43 performs various kinds of necessary image processing such as gradation processing on the ultrasound image signal input from the DSC 42, and then outputs the signal representing the ultrasound image to the display controller 22 and the image memory 24. The signal representing the ultrasound image generated by the image generation unit 21 in this manner is simply referred to as an ultrasound image.

The transmission and reception circuit 12 of the ultrasound probe 10 and the image generation unit 21 of the apparatus main body 20 form an image acquisition unit 32 that acquires a plurality of ultrasound images in which the lesion part of the subject is imaged.

The image memory 24 is a memory that stores the ultrasound image generated by the image acquisition unit 32 under the control of the main body controller 29. For example, the image memory 24 can hold the ultrasound images of a plurality of frames, which are generated by the image acquisition unit 32, corresponding to the diagnosis for the breast of the subject.

Here, as the image memory 24, recording media such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory), or the like can be used.

The analysis unit 25 analyzes the plurality of ultrasound images that are generated by the image acquisition unit 32 and stored in the image memory 24 to acquire probe positional information indicating the position of the ultrasound probe 10, image type information indicating whether the ultrasound image is a B-mode image or another image, and imaging feature information regarding the position of the imaged lesion part and the imaging range, for each of the plurality of ultrasound images.

The analysis unit 25 can acquire the probe positional information on the basis of the orientation and the probe position of a body mark added to each of the plurality of ultrasound images.

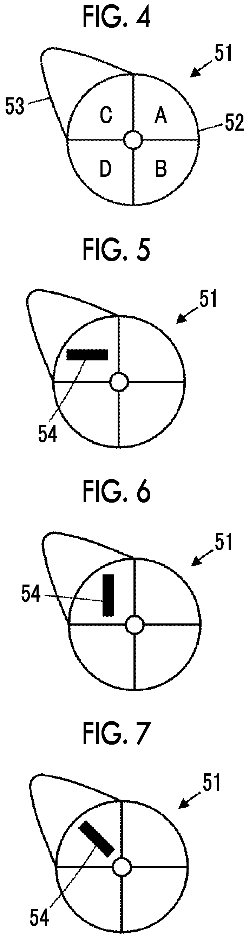

Fig. 4 illustrates a body mark 51 schematically representing the right breast viewed from the front, as an example of the body mark. The body mark 51 has a circular breast region 52, and a substantially triangular axillary region 53 representing the armpit and extending obliquely upward from the breast region 52. The breast region 52 is divided into four regions of an inner upper region A, an inner lower region B, an outer upper region C, and an outer lower region D of the breast, and the axillary region 53 is connected to a left oblique upper portion of the outer upper region C.

A body mark schematically representing the left breast is obtained by horizontally reversing the body mark 51 illustrated in Fig. 4.

As illustrated in Fig. 5, a probe mark 54 indicating the position and orientation of the ultrasound probe 10 in a case of capturing the ultrasound image is plotted on the body mark 51. The probe mark 54 can be plotted on the body mark 51, for example, by the user manually operating the input device 30, or a sensor that detects the position and orientation of the ultrasound probe 10 is provided, and the probe mark 54 can be automatically plotted on the body mark 51 on the basis of the detection signal output from the sensor.

The probe mark 54 has a rectangular shape extending in an elongated manner in an arrangement direction of the transducer array 11 of the ultrasound probe 10, and it is possible to understand the position and orientation of the ultrasound probe 10 at the time of imaging, by checking the probe mark 54 plotted on the body mark 51. For example, in a case where the body mark 51 illustrated in Fig. 5 is added to the ultrasound image, it can be seen that the image is captured by positioning the ultrasound probe 10 at the outer upper region of the right breast in an orientation along a horizontal direction (shoulder width direction of the subject).

Further, in a case where the body mark 51 illustrated in Fig. 6 is added to the ultrasound image, it can be seen that the image is captured by positioning the ultrasound probe 10 at the outer upper region of the right breast in a vertical direction (orientation along a center line extending from the head toward the feet of the subject).

Furthermore, in a case where the body mark 51 illustrated in Fig. 7 is added to the ultrasound image, it can be seen that the image is captured by positioning the ultrasound probe 10 at the outer upper region of the right breast in a diagonal orientation from the right armpit to the nipple of the right breast of the subject instead of being in the horizontal direction or vertical direction.

Therefore, the analysis unit 25 can perform the image analysis on the ultrasound image to recognize the probe mark 54 of the body mark 51 added to the ultrasound image, and acquire the probe positional information regarding the position and orientation of the ultrasound probe 10 at the time of imaging the ultrasound image.

In the image memory 24 in which the ultrasound image is stored, tag information associated with the image is stored as accessory information of the ultrasound image, and in a case of being transferred from the apparatus main body 20 to a picture archiving and communication system (PACS), a workstation, or the like, the ultrasound image is converted into the image data in a Digital Imaging and Communications In Medicine (DICOM) format including the tag information.

The tag information includes, for example, that the ultrasound image is monochrome. In a case where the ultrasound image is monochrome, it can be determined that the ultrasound image is a B-mode image. Thus, the analysis unit 25 can acquire the image type information indicating whether the ultrasound image is a B-mode image or another image on the basis of the tag information associated with each of the plurality of ultrasound images.

The analysis unit 25 can perform the image analysis on the ultrasound image to recognize RGB signal values of the ultrasound image, and acquire the image type information of the ultrasound image. In a case where it is determined that the ultrasound image is a grayscale image on the basis of the RGB signal values in each pixel of the ultrasound image, it can be determined that the ultrasound image is a B-mode image.

Further, the analysis unit 25 may acquire the image type information of the ultrasound image on the basis of the operation mode of the ultrasound device at the time of imaging the ultrasound image. For example, in a case where the ultrasound device is in an elasticity mode during freezing, it can be determined that the ultrasound image is an image superimposed with the elasticity information.

For example, since breast cancer is generated from the epithelium of the breast duct, the continuity of the breast ducts extending from the mammary gland to the nipple is observed, a site where the continuity is interrupted is often extracted as the lesion part, and the user such as an examiner may want to leave an image in which not only the lesion part but also the breast duct leading to the lesion part are imaged.

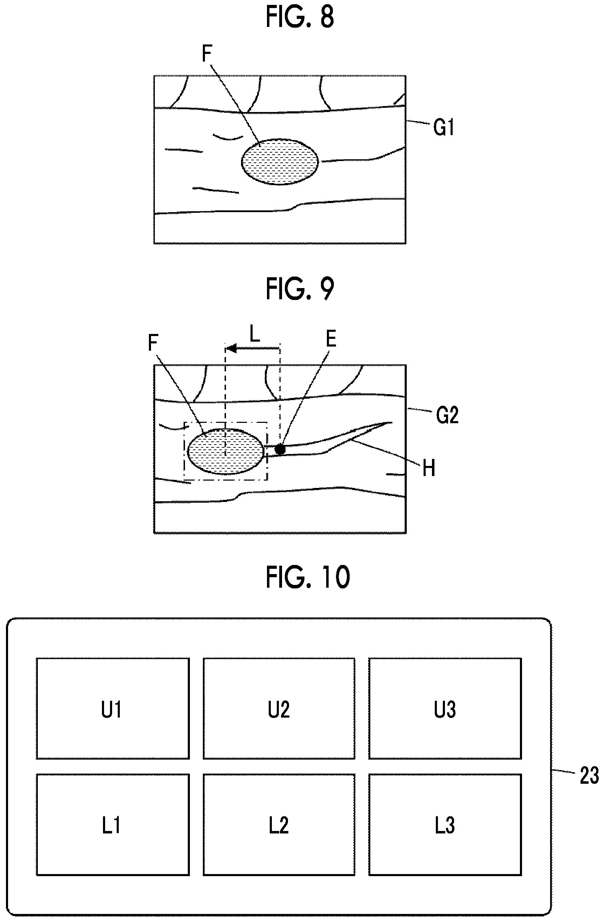

In this case, by setting a lesion part F at a biased position toward the end portion of the screen as illustrated in Fig. 9 with respect to an ultrasound image G1 captured by positioning the lesion part F near the center of the screen as illustrated in Fig. 8, an ultrasound image G2 in which not only the lesion part F but also a breast duct H are imaged can be obtained.

Therefore, the analysis unit 25 performs the image analysis on each of the plurality of ultrasound images to detect the lesion part from the ultrasound image and acquire the imaging feature information regarding the position of the lesion part in the ultrasound image. For example, as illustrated in Fig. 9, the analysis unit 25 can calculate a distance L between a center E of the ultrasound image G2 and the lesion part F in the horizontal direction, and understand the position of the lesion part F in the horizontal direction on the basis of the calculated distance L.

As the position of the lesion part in the ultrasound image, for example, three regions near the center of the ultrasound image in the horizontal direction (orientation direction), near the end portion of the ultrasound image in the horizontal direction, and near the middle of the center and the end portion of the ultrasound image in the horizontal direction are assumed, and it is determined which region the lesion part F is positioned on the basis of the calculated distance L. The analysis unit 25 can acquire the imaging feature information indicating the region where the lesion part is positioned for each of the plurality of ultrasound images.

The analysis unit 25 acquires the imaging feature information regarding the imaging range of the inside of the subject shown in the ultrasound image on the basis of the distance between pixels in each of the plurality of ultrasound images. As the distance of pixel is greater, the imaging is performed over a wider range, and for example, it can be seen that not only the lesion part but also the peripheral tissues of the lesion part are imaged. Since the distance between pixels is stored in a tag attached to the image data in the DICOM format, it is possible to acquire the distance between pixels in the ultrasound image as the imaging feature information by checking the tag.

The analysis of the ultrasound image by the analysis unit 25 can be executed by using at least one of template matching, an image analysis technique using feature amounts such as Adaptive Boosting (Adaboost), support vector machines (SVM) or scale-invariant feature transform (SIFT), and a determination model trained using a machine learning technique such as deep learning.

The information addition unit 26 adds the probe positional information, the image type information, and the imaging feature information acquired by the analysis unit 25 to each of the plurality of ultrasound images. The plurality of ultrasound images to which the probe positional information, the image type information, and the imaging feature information are added are sent from the information addition unit 26 to the image memory 24 to be stored in the image memory 24, and are sent from the information addition unit 26 to the image extraction unit 27. The addition of the probe positional information, the image type information, and the imaging feature information can be performed on the plurality of ultrasound images stored in the image memory 24, and performed in the DICOM file of the plurality of ultrasound images.

The image extraction unit 27 extracts the ultrasound image that conforms to the display layout set by the display layout setting unit 28, from the plurality of ultrasound images by referring to the probe positional information, the image type information, and the imaging feature information acquired by the analysis unit 25.

The image extraction unit 27 can first extract only the B-mode images from the plurality of ultrasound images on the basis of the image type information acquired by the analysis unit 25, and select and extract an image conforming to the display layout from the B-mode images.

In a case where the display layout set by the display layout setting unit 28 is a layout including not only the B-mode image but also an image superimposed with the elasticity information or Doppler information, the image extraction unit 27 can extract an image conforming to the display layout including the B-mode image and the image superimposed with the elasticity information or Doppler information, from the plurality of ultrasound images.

The display layout setting unit 28 sets the display layout in a case of displaying the plurality of ultrasound images on the monitor 23, on the basis of the user's input operation via the input device 30. The display layout is set by the user such that the plurality of ultrasound images of the types and imaging features according to the preference of each user are displayed on the monitor 23 at the same time, and the number of screen divisions of the monitor 23, the display order corresponding to the image types and imaging features, the display magnification, and the like can be freely specified.

Fig. 10 illustrates an example of the display layout set by the display layout setting unit 28. The display layout is obtained by dividing the screen of the monitor 23 into six display regions of display regions U1 to U3 and L1 to L3, and the plurality of ultrasound images of which all the image types are B-mode images are displayed in the display regions U1 to U3, and L1 to L3 with the same display magnification.

For example, images captured by the ultrasound probe 10 in the orientation along the horizontal direction are displayed in the upper display regions U1 to U3, and images captured by the ultrasound probe 10 in the orientation along the vertical direction are displayed in the lower display regions L1 to L3.

Images in which the lesion part is shown near the center in the horizontal direction are displayed in the upper and lower left display regions U1 and L1, images in which the lesion part is shown near the middle of the center and the end portion in the horizontal direction are displayed in the upper and lower middle display regions U2 and L2, and images in which the lesion part is shown near the end portion in the horizontal direction are displayed in the upper and lower right display regions U3 and L3.

The display controller 22 performs predetermined processing on the ultrasound image sent from the image generation unit 21, and displays the ultrasound image on the monitor 23, under the control of the main body controller 29.

The display controller 22 displays the ultrasound image extracted by the image extraction unit 27 on the monitor 23 according to the display layout set by the display layout setting unit 28.

The monitor 23 is for displaying the ultrasound image under the control of the display controller 22, and includes a display device such as a liquid crystal display (LCD), or an organic electroluminescence (EL) display.

The main body controller 29 controls each unit of the apparatus main body 20 and the transmission and reception circuit 12 of the ultrasound probe 10 on the basis of a control program and the like stored in advance.

Although not illustrated, a main body-side storage unit is connected to the main body controller 29. The main body-side storage unit stores a control program and the. As the main body-side storage unit, for example, a flash memory, a RAM, an SD card, an SSD, and the like can be used.

The input device 30 is for a user to perform an input operation, and is configured by, for example, a device such as a keyboard, a mouse, a trackball, a touchpad, and a touch sensor superimposed on the monitor 23.

The processor 31 having the image generation unit 21, the display controller 22, the analysis unit 25, the information addition unit 26, the image extraction unit 27, the display layout setting unit 28, and the main body controller 29 is configured by a central processing unit (CPU) and a control program for causing the CPU to execute various kinds of processing, but the processor 31 may be configured by using a field programmable gate array (FPGA), a digital signal processor (DSP), an application specific integrated circuit (ASIC), a graphics processing unit (GPU), or other integrated circuits (IC) or may be configured by a combination thereof.

Further, the image generation unit 21, the display controller 22, the analysis unit 25, the information addition unit 26, the image extraction unit 27, the display layout setting unit 28, the main body controller 29 of the processor 31 can also be configured by being integrated partially or entirely into one CPU or the like.

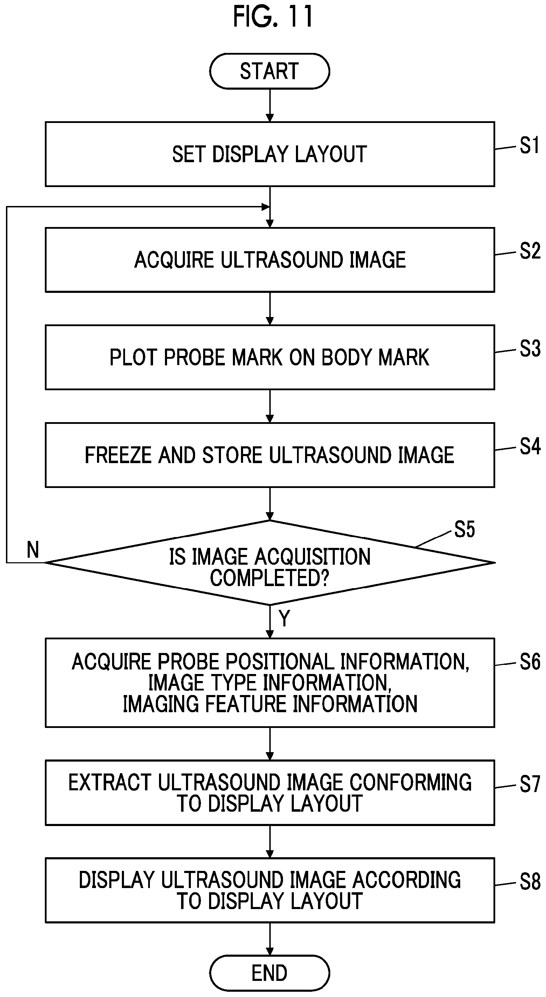

Next, the operation of the image display apparatus according to the first embodiment will be described with reference to the flowchart illustrated in Fig. 11.

First, in Step S1, the display layout is set by the display layout setting unit 28 on the basis of the user's input operation via the input device 30. The user can set the display layout as illustrated in Fig. 10 in which the number of screen divisions of the monitor 23, the display order corresponding to the image types and imaging features, and the display magnification are defined, according to his or her own preference.

The display layout is sent from the display layout setting unit 28 to the image extraction unit 27 and the display controller 22.

Next, in Step S2, the ultrasound image in which the lesion part of the subject is imaged is acquired by the image acquisition unit 32. In this case, under the control of the main body controller 29, the transmission and reception of ultrasonic waves from the plurality of transducers of the transducer array 11 are started according to the drive signal from the pulser 13 of the transmission and reception circuit 12 of the ultrasound probe 10, the ultrasound echo from the subject is received by the plurality of transducers of the transducer array 11, and the reception signal as the analog signal is output to the amplification unit 14 to be amplified, and then is subjected to the AD conversion by the AD conversion unit 15 to acquire the reception data.

By performing the reception focusing processing on the reception data by the beam former 16, the sound ray signal generated in this manner is sent to the image generation unit 21 of the apparatus main body 20, and the ultrasound image representing the tomographic image information of the lesion part of the subject is generated by the image generation unit 21. In this case, the signal processing unit 41 of the image generation unit 21 performs the correction of the attenuation according to the depth of the reflection position of the ultrasonic wave and the envelope detection processing on the sound ray signal, the DSC 42 performs the conversion into the image signal according to a normal television signal scanning method, and the image processing unit 43 performs various kinds of necessary image processing such as gradation processing.

In a case where the ultrasound image is acquired by the image acquisition unit 32, in Step S3, the probe mark is plotted on the body mark automatically or manually by the user, and then in Step S4, the ultrasound image is frozen and stored in the image memory 24. In this case, the tag information associated with the ultrasound image as the accessory information of the ultrasound image is also stored in the image memory 24.

Until it is determined in Step S5 that the acquisition of the series of ultrasound images in the ultrasonography of the subject is completed, Step S2 to Step S5 are repeated, and the plurality of acquired ultrasound images are stored in the image memory 24.

In a case where it is determined in Step S5 that the acquisition of the series of ultrasound images is completed, the processing proceeds to Step S6, and the analysis is performed on the plurality of ultrasound images stored in the image memory 24 by the analysis unit 25. Thus, the probe positional information indicating the position of the ultrasound probe 10 at the time of imaging, the image type information indicating whether the ultrasound image is a B-mode image or another image, and the imaging feature information regarding the position of the imaged lesion part and the imaging range are acquired for each of the plurality of ultrasound images.

In this case, for example, the analysis unit 25 acquires the probe positional information from the tag information that is stored in Step S4 in the image memory 24 as the accessory information of the ultrasound image.

Further, the analysis unit 25 acquires the image type information on the basis of the tag information stored in association with the ultrasound image in the image memory 24 or by recognizing the RGB signal values of the ultrasound image. Furthermore, the analysis unit 25 may acquire the image type information of the ultrasound image on the basis of the operation mode of the ultrasound device at the time of imaging the ultrasound image. For example, in a case where the ultrasound device is in the elasticity mode during freezing, the image type information indicating an image superimposed with the elasticity information can be acquired.

The analysis unit 25 performs the image analysis on the ultrasound image to detect the lesion part, acquire the imaging feature information regarding the position of the lesion part in the ultrasound image, and acquire the imaging feature information regarding the imaging range on the basis of the distance between pixels in the ultrasound image.

The probe positional information, the image type information, and the imaging feature information acquired by the analysis unit 25 are added to the corresponding ultrasound image by the information addition unit 26. The plurality of ultrasound images in which probe positional information, the image type information, and the imaging feature information are added are sent from the information addition unit 26 to the image extraction unit 27, and are sent to and stored in the image memory 24.

In subsequent Step S7, the ultrasound image conforming to the display layout set by the display layout setting unit 28 is extracted from the plurality of ultrasound images by the image extraction unit 27.

In this case, the image extraction unit 27 extracts the ultrasound image by referring to the probe positional information, the image type information, and the imaging feature information acquired by the analysis unit 25. For example, the ultrasound image with the probe positional information indicating that the image is captured by the ultrasound probe 10 in an orientation along the horizontal direction at the position of the lesion part of interest, the image type information indicting the B-mode image, and the imaging feature information indicating that the lesion part is shown near the center in the horizontal direction is extracted corresponding to the display region U1 of the display layout illustrated in Fig. 10.

Similarly, the ultrasound image conforming to each of the plurality of display regions of the display layout set by the display layout setting unit 28 is extracted by the image extraction unit 27.

Further, in Step S8, the plurality of ultrasound images extracted in Step S7 are displayed on the monitor 23 according to the display layout by the display controller 22.

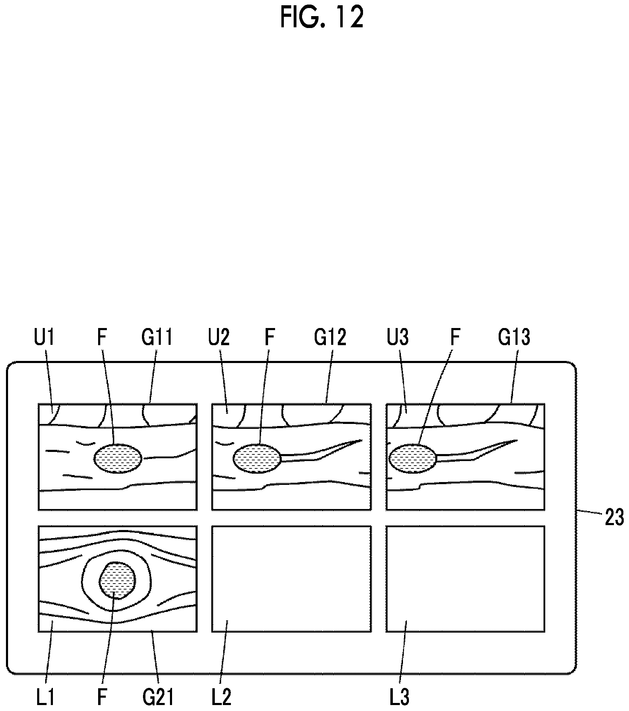

Fig. 12 illustrates an example of the plurality of ultrasound images displayed on the monitor 23 according to the display layout illustrated in Fig. 10. In the upper display regions U1 to U3, three ultrasound images G11 to G13 captured by the ultrasound probe 10 in an orientation along the horizontal direction are displayed, respectively. In the lower display region L1, an ultrasound image G21 captured by the ultrasound probe 10 in an orientation along the vertical direction is displayed.

The lesion part F is shown near the center in the horizontal direction in the ultrasound images G11 and G21 displayed in the upper and lower left display regions U1 and L1, the lesion part F is shown near the middle between the center and the end portion in the horizontal direction in the ultrasound image G12 displayed in the upper center display region U2, and the lesion part F is shown near the end portion in the horizontal direction in the ultrasound image G13 displayed in the upper right display region U3.

In Fig. 12, in the ultrasound images G11 to G13 displayed in the upper display regions U1 to U3, the breast duct leading to the lesion part F is imaged as the position of the lesion part F is shifted in the horizontal direction, and therefore, three ultrasound images G11 to G13 are displayed. However, in Fig. 12, in the ultrasound image G21 displayed in the lower display region L1, the breast duct leading to the lesion part F is not imaged, there is little need to display an image in which the position of the lesion part F is simply shifted in the horizontal direction, and therefore, ultrasound images are not displayed in the lower display regions L2 and L3.

One of the image captured by the ultrasound probe 10 in an orientation along the horizontal direction, which is displayed in the upper display regions U1 to U3, and the image captured by the ultrasound probe 10 in an orientation along the vertical direction, which is displayed in the lower display regions L1 to L3, can be generated from a video imaged at the time of acquiring the other image.

In this manner, the probe positional information of the ultrasound probe 10, the image type information indicating whether the image is the B-mode image or another image, and the imaging feature information regarding the position of the lesion part and the imaging range are acquired by the analysis unit 25 for each of the plurality of ultrasound images, the ultrasound image conforming to the display layout set by the display layout setting unit 28 is extracted from the plurality of ultrasound images by the image extraction unit 27 on the basis of the probe positional information, the image type information, and the imaging feature information, and the extracted ultrasound image is displayed on the monitor 23 according to the display layout by the display controller 22. Therefore, it is possible for the user to efficiently perform the interpretation by checking the ultrasound image displayed on the monitor 23.

Since the image extraction unit 27 refers to the probe positional information indicating the position and orientation of the ultrasound probe 10 at the time of imaging, and the image type information, the image extraction unit 27 can extract a tomographic B-mode image of the same lesion part cut in the same cross-sectional direction.

Further, since the image extraction unit 27 refers to the imaging feature information regarding the position of the lesion part in the ultrasound image, the image extraction unit 27 can extract the ultrasound image in which not only the lesion part but also the peripheral portion leading to the lesion part are imaged.

Since the analysis unit 25 acquires the imaging feature information regarding the imaging range of the inside of the subject shown in the ultrasound image on the basis of the distance between pixels in each of the plurality of ultrasound images, the display layout for displaying the plurality of ultrasound images, in which the same lesion part is imaged but which have different imaging ranges, can be set by the display layout setting unit 28.

In a case where the display layout including not only the B-mode image but also the image superimposed with the elasticity information or Doppler information is set by the display layout setting unit 28, the image superimposed with the elasticity information or the image superimposed with the Doppler information can be displayed on the monitor 23 together with the B-mode image at the same time.

### Second Embodiment

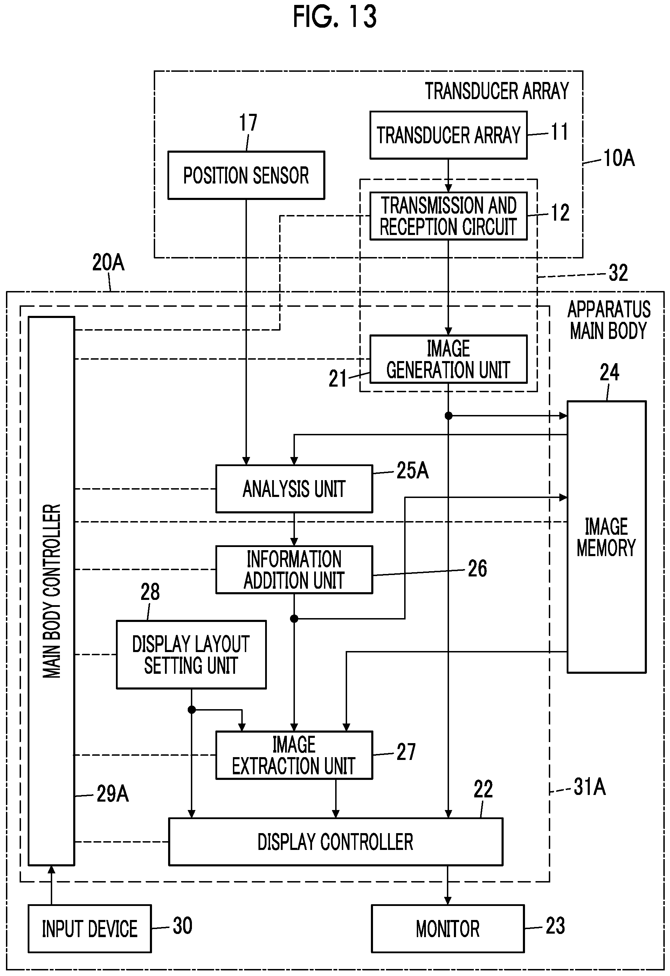

Fig. 13 illustrates a configuration of an image display apparatus according to a second embodiment. The image display apparatus has a function as an ultrasound device that acquires an ultrasound image, and includes an ultrasound probe 10A and an apparatus main body 20A that are connected to each other. The ultrasound probe 10A has a position sensor 17 in addition to the transducer array 11 and the transmission and reception circuit 12 in the ultrasound probe 10 in the first embodiment. the apparatus main body 20A uses an analysis unit 25A and a main body controller 29A instead of the analysis unit 25 and the main body controller 29 in the apparatus main body 20 in the first embodiment, and the other configuration thereof is the same as the apparatus main body 20 in the first embodiment.

The position sensor 17 of the ultrasound probe 10A is connected to the analysis unit 25A of the apparatus main body 20A, and the analysis unit 25A is connected to the image memory 24 and the information addition unit 26.

A main body controller 29A is connected to the image generation unit 21, the display controller 22, the image memory 24, the analysis unit 25A, the information addition unit 26, the image extraction unit 27, and the display layout setting unit 28, and the input device 30 is connected to the main body controller 29A. The transmission and reception circuit 12 of the ultrasound probe 10 is connected to the main body controller 29A.

The image generation unit 21, the display controller 22, the analysis unit 25A, the information addition unit 26, the image extraction unit 27, the display layout setting unit 28, and the main body controller 29A constitute a processor 31A.

The position sensor 17 of the ultrasound probe 10A detects the position and orientation of the ultrasound probe 10A, and sends the position and orientation of the ultrasound probe 10A to the analysis unit 25A of the apparatus main body 20A. For example, as the position sensor 17, a magnetic sensor, an optical position sensor, an acceleration sensor, a gyro sensor, or a global positioning system (GPS) sensor can be used.

The analysis unit 25A acquires the probe positional information on the basis of the position and orientation of the ultrasound probe 10A detected by the position sensor 17. Therefore, for example, even in a case where the body mark 51 is not added to the ultrasound image, the probe positional information regarding the position and orientation of the ultrasound probe 10 can be acquired.

The image type information indicating whether the ultrasound image is the B-mode image or another image, and the imaging feature information regarding the region where the lesion part is positioned and the imaging range are acquired in the same manner as in the analysis unit 25 in the first embodiment.

The probe positional information, the image type information, and the imaging feature information acquired by the analysis unit 25A are added to the ultrasound image by the information addition unit 26, and the image extraction unit 27 extracts the ultrasound image by referring to the probe positional information, the image type information, and the imaging feature information. Therefore, as in the first embodiment, the ultrasound image extracted by the image extraction unit 27 is displayed on the monitor 23 according to the display layout, and it is possible for the user to efficiently perform the interpretation by checking the ultrasound image displayed on the monitor 23.

### Third Embodiment

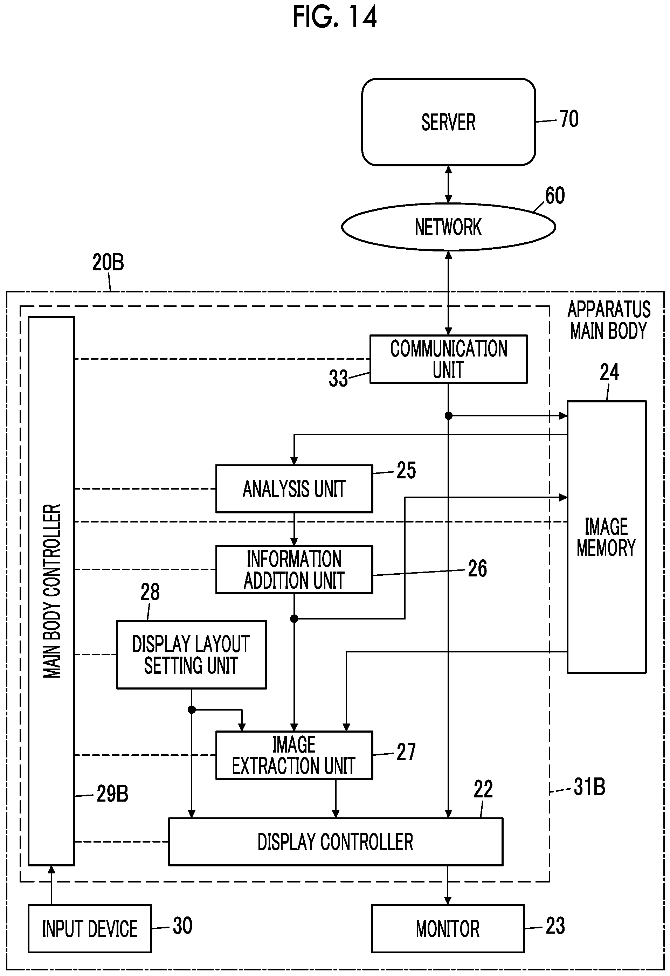

Fig. 14 illustrates a configuration of an image display apparatus according to a third embodiment. Unlike the first and second embodiments, the image display apparatus does not have a function as an ultrasound device that acquires the ultrasound image, and has a configuration in which an apparatus main body 20B instead of the apparatus main body 20 is connected to the ultrasound probe 10 in the image display apparatus of the first embodiment illustrated in Fig. 1. the apparatus main body 20B has a communication unit 33 instead of the image generation unit 21 and uses a main body controller 29B instead of the main body controller 29 in the apparatus main body 20 in the first embodiment, and the other configuration thereof is the same as the apparatus main body 20 in the first embodiment.

The communication unit 33 is connected to the image memory 24, and is connected to a server 70 via a network 60.

The main body controller 29B is connected to the display controller 22, the image memory 24, the analysis unit 25, the information addition unit 26, the image extraction unit 27, the display layout setting unit 28, and the communication unit 33, and the input device 30 is connected to the main body controller 29B. The transmission and reception circuit 12 of the ultrasound probe 10 is connected to the main body controller 29B.

The display controller 22, the analysis unit 25, the information addition unit 26, the image extraction unit 27, the display layout setting unit 28, the main body controller 29B, and the communication unit 33 constitute a processor 31B.

For example, the server 70 is installed in a facility such as a hospital, and is connected to the communication unit 33 of the apparatus main body 20B via the network 60. The server 70 manages image data of the ultrasound image and the like acquired by the ultrasonography for the subject, and can be used as a server in a PACS or workstation. The ultrasound image stored in the server 70 is acquired by the ultrasound device, and then converted into the image data in a DICOM format including the tag information in a case of being transferred from the ultrasound device.

The communication unit 33 is configured by a circuit including an antenna for transmitting and receiving radio waves, and a circuit or the like for performing local area network (LAN) connection, and performs communication with the server 70 via the network 60 under the control of the main body controller 29B. The communication unit 33 can receive the ultrasound image from the server 70 via the network 60.

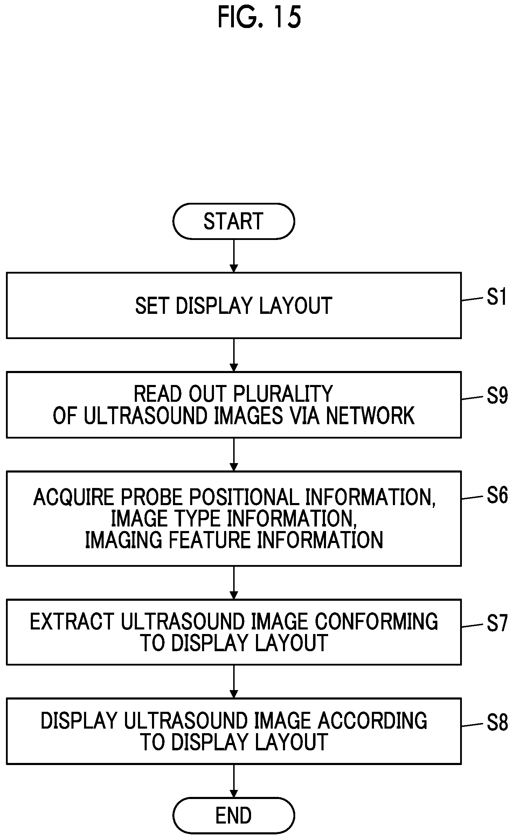

The operation of the image display apparatus according to the third embodiment will be described with reference to the flowchart illustrated in Fig. 15.

As in the first embodiment, first, in Step S1, the display layout is set by the display layout setting unit 28 on the basis of the user's input operation via the input device 30.

Next, in Step S9, on the basis of the user's input operation via the input device 30, the ultrasound image of the subject stored in the server 70 is transmitted to the communication unit 33 via the network 60, and is stored in the image memory 24.

Subsequent Step S6 to Step S8 are the same as those in the first embodiment. That is, in Step S6, the analysis unit 25 reads out the plurality of ultrasound images of the subject from the image memory 24, and acquires the probe positional information indicating the position of the ultrasound probe 10 at the time of imaging, the image type information indicating whether the ultrasound image is a B-mode image or another image, and the imaging feature information regarding the position of the imaged lesion part and the imaging range, for each ultrasound image. In this case, the analysis unit 25 can acquire the probe positional information by recognizing the probe mark of the body mark added to the ultrasound image. The ultrasound image transmitted to the image memory 24 from the server 70 via the network 60 has already been converted into the image data in the DICOM format including the tag information, the analysis unit 25 can acquire the image type information of the ultrasound image on the basis of the tag information included in the DICOM file.

The acquired probe positional information, image type information, and imaging feature information are added to the ultrasound image by the information addition unit 26, and in Step S7, the image extraction unit 27 extracts the ultrasound image by referring to the probe positional information, the image type information, and the imaging feature information. Thereafter, in Step S8, the plurality of ultrasound images extracted in Step S7 are displayed on the monitor 23 according to the display layout.

Therefore, as in the first and second embodiments, the ultrasound image extracted by the image extraction unit 27 is displayed on the monitor 23 according to the display layout, and it is possible for the user to efficiently perform the interpretation by checking the ultrasound image displayed on the monitor 23.

Therefore, for example, the plurality of ultrasound images that are acquired by the past examination and are stored in the server 70 are read out, and displayed according to the display layout.

As described above, the analysis unit 25 acquires the image type information of the ultrasound image on the basis of the tag information included in the DICOM file, the image display apparatus according to the third embodiment can be used as a viewer on the PACS, for example.

In the first to third embodiments described above, in a case where there are a plurality of lesion parts F, the display layout as illustrated in Fig. 10 is switched and displayed on the monitor 23 for each lesion part F.

The connection method of the ultrasound probes 10 and 10A with the apparatus main bodies 20 and 20A in the first and second embodiments described above is not particularly limited, and may be wired connection or wireless connection.

In the first and second embodiments described above, the ultrasound probes 10 and 10A have the transmission and reception circuit 12, but the apparatus main bodies 20 and 20a can include the transmission and reception circuit 12. Further, the apparatus main bodies 20 and 20Ahave the image generation unit 21, but the ultrasound probes 10 and 10A may have the image generation unit 21. Further, among the signal processing unit 41, the DSC 42, and the image processing unit 43 constituting the image generation unit 21 illustrated in Fig. 3, only the signal processing unit 41 may be provided in the ultrasound probes 10 and 10A, and the apparatus main bodies 20 and 20A may have the DSC 42 and the image processing unit 43.

As the apparatus main bodies 20, 20A, and 20B in the first to third embodiments, a stationary type apparatus main body can be used, and a portable or handheld type compact apparatus main body can also be used.

### Explanation of References

10: ultrasound probe
11: transducer array
12: transmission and reception circuit
13: pulser
14: amplification unit
15: AD conversion unit
16: beam former
17: position sensor
20, 20A, 20B: apparatus main body
21: image generation unit
22: display controller
23: monitor
24: image memory
25, 25A: analysis unit
26: information addition unit
27: image extraction unit
28: display layout setting unit
29, 29A, 29B: main body controller
30: input device
31, 31A, 31B: processor
32: image acquisition unit
33: communication unit
41: signal processing unit
42: DSC
43: image processing unit
51: body mark
52: breast region
53: axillary region
54: probe mark
60: network
70: server
G1, G2, G11, G12, G13, G21, G21, G22, G23: ultrasound image
F: lesion part
E: center
L: distance
H: breast duct
U1, U2, U3, L1, L2, L3: display region

## Claims

1. An image display apparatus comprising:
a monitor (23);
an analysis unit (25, 25A) that analyzes a plurality of ultrasound images in which a lesion part (F) of a subject is imaged to acquire probe positional information of an ultrasound probe (10) at a time of imaging, and imaging feature information regarding a position of the lesion part and an imaging range on the basis of a distance between pixels in each of the plurality of ultrasound images;
an image extraction unit (27) that extracts an ultrasound image conforming to a display layout set by a user, from the plurality of ultrasound images by referring to the probe positional information and the imaging feature information acquired by the analysis unit; and
a display controller (22) that displays the ultrasound image extracted by the image extraction unit on the monitor according to the display layout.

2. The image display apparatus according to claim 1, further comprising:
an ultrasound probe (10); and
an image acquisition unit (32) that acquires the plurality of ultrasound images in which the lesion part of the subject is imaged by using the ultrasound probe,
wherein the analysis unit analyzes the plurality of ultrasound images acquired by the image acquisition unit.

3. The image display apparatus according to claim 1 or 2, further comprising:
an input device (30) for the user to perform an input operation; and
a display layout setting unit (28) that sets the display layout on the basis of the user's input operation via the input device.

4. The image display apparatus according to claim 1 or 2, further comprising:
an information addition unit (26) that adds the probe positional information and the imaging feature information acquired by the analysis unit to each of the plurality of ultrasound images,
wherein the image extraction unit extracts the ultrasound image by referring to the probe positional information and the imaging feature information added to the plurality of ultrasound images.

5. The image display apparatus according to claim 1 or 2,
wherein the analysis unit (25, 25A) acquires the probe positional information on the basis of a probe position and an orientation of a body mark (51) added to each of the plurality of ultrasound images.

6. The image display apparatus according to claim 2,
wherein the ultrasound probe (10) has a position sensor (17), and
the analysis unit (25, 25A) acquires the probe positional information on the basis of a probe position detected by the position sensor.

7. The image display apparatus according to claim 1 or 2,
wherein the analysis unit (25, 25A) performs image analysis on each of the plurality of ultrasound images to detect the lesion part and acquire the imaging feature information regarding the position of the lesion part.

8. The image display apparatus according to claim 1 or 2,
wherein the analysis unit (25, 25A) acquires image type information indicating whether the ultrasound image is a B-mode image or another image, together with the probe positional information and the imaging feature information, and
the image extraction unit (27) extracts the ultrasound image by referring to the probe positional information, the image type information, and the imaging feature information that are acquired by the analysis unit.

9. The image display apparatus according to claim 8,
wherein the analysis unit (25, 25A) acquires the image type information on the basis of tag information added to each of the plurality of ultrasound images.

10. The image display apparatus according to claim 8,
wherein the analysis unit (25, 25A) acquires the image type information on the basis of RGB signal values of each of the plurality of ultrasound images.

11. The image display apparatus according to claim 8,
wherein the image extraction unit (27) extracts a B-mode image from the plurality of ultrasound images on the basis of the image type information.

12. A control method of an image display apparatus, the control method comprising:
inputting a plurality of ultrasound images in which a lesion part of a subject is imaged;
analyzing the plurality of ultrasound images to acquire probe positional information of an ultrasound probe, and imaging feature information regarding a position of the lesion part and an imaging range on the basis of a distance between pixels in each of the plurality of ultrasound images;
extracting an ultrasound image conforming to a display layout set by a user, from the plurality of ultrasound images by referring to the acquired probe positional information and the acquired imaging feature information; and
displaying the extracted ultrasound image on a monitor according to the display layout.

## Patentansprüche

1. Bildanzeigevorrichtung, umfassend:
einen Monitor (23);
eine Analyseeinheit (25, 25A), die mehrere Ultraschallbilder, in denen ein Läsionsteil (F) einer Untersuchungsperson abgebildet ist, analysiert, um Sondenpositionsinformationen einer Ultraschallsonde (10) zu einem Zeitpunkt von Bildgebung und Bildgebungsmerkmalsinformationen in Bezug auf eine Position des Läsionsteils und einen Bildgebungsbereich auf der Grundlage einer Entfernung zwischen Pixeln in jedem der mehreren Ultraschallbilder zu erfassen;
eine Bildextraktionseinheit (27), die ein Ultraschallbild, das mit einem von einem Benutzer eingestellten Anzeigelayout konform geht, aus den mehreren Ultraschallbildern unter Bezugnahme auf die Sondenpositionsinformationen und die von der Analyseeinheit erfassten Bildgebungsmerkmalsinformationen extrahiert; und
eine Anzeigesteuerung (22), die das von der Bildextraktionseinheit extrahierte Ultraschallbild auf dem Monitor gemäß dem Anzeigelayout anzeigt.

2. Bildanzeigevorrichtung nach Anspruch 1, ferner umfassend:
eine Ultraschallsonde (10); und
eine Bilderfassungseinheit (32), die die mehreren Ultraschallbilder, in denen der Läsionsteil der Untersuchungsperson abgebildet ist, durch Verwenden der Ultraschallsonde erfasst,
wobei die Analyseeinheit die mehreren Ultraschallbilder, die von der Bilderfassungseinheit erfasst werden, analysiert.

3. Bildanzeigevorrichtung nach Anspruch 1 oder 2, ferner umfassend:
eine Eingabevorrichtung (30), durch die der Benutzer eine Eingabebedienung durchführt; und
eine Anzeigelayout-Einstelleinheit (28), die das Anzeigelayout auf der Grundlage der Eingabebedienung seitens von Benutzer via die Eingabevorrichtung einstellt.

4. Bildanzeigevorrichtung nach Anspruch 1 oder 2, ferner umfassend:
eine Informationshinzufügungseinheit (26), die die Sondenpositionsinformationen und die von der Analyseeinheit erfassten Bildgebungsmerkmalsinformationen zu jedem der mehreren Ultraschallbilder hinzufügt,
wobei die Bildextraktionseinheit das Ultraschallbild unter Bezugnahme auf die Sondenpositionsinformationen und die Bildgebungsmerkmalsinformationen, die zu den mehreren Ultraschallbildern hinzugefügt wurden, extrahiert.

5. Bildanzeigevorrichtung nach Anspruch 1 oder 2,
wobei die Analyseeinheit (25, 25A) die Sondenpositionsinformationen auf der Grundlage einer Sondenposition und einer Ausrichtung einer Körpermarkierung (51), die zu jedem der mehreren Ultraschallbilder hinzugefügt wurde, erfasst.

6. Bildanzeigevorrichtung nach Anspruch 2,
wobei die Ultraschallsonde (10) einen Positionssensor (17) aufweist, und die Analyseeinheit (25, 25A) die Sondenpositionsinformationen auf der Grundlage einer von dem Positionssensor detektierten Sondenposition erfasst.

7. Bildanzeigevorrichtung nach Anspruch 1 oder 2,
wobei die Analyseeinheit (25, 25A) Bildanalyse an jedem der mehreren Ultraschallbilder durchführt, um den Läsionsteil zu detektieren und die Bildgebungsmerkmalsinformationen in Bezug auf die Position des Läsionsteils zu erfassen.

8. Bildanzeigevorrichtung nach Anspruch 1 oder 2,
wobei die Analyseeinheit (25, 25A) Bildtypinformationen, die angeben, ob das Ultraschallbild ein B-Modus-Bild oder ein anderes Bild ist, zusammen mit den Sondenpositionsinformationen und den Bildgebungsmerkmalsinformationen erfasst, und
die Bildextraktionseinheit (27) das Ultraschallbild durch Bezugnahme auf die Sondenpositionsinformationen, die Bildtypinformationen und die Bildgebungsmerkmalsinformationen, die von der Analyseeinheit erfasst werden, extrahiert.

9. Bildanzeigevorrichtung nach Anspruch 8,
wobei die Analyseeinheit (25, 25A) die Bildtypinformationen auf der Grundlage von Etiketteninformationen, die zu jedem der mehreren Ultraschallbilder hinzugefügt werden, erfasst.

10. Bildanzeigevorrichtung nach Anspruch 8,
wobei die Analyseeinheit (25, 25A) die Bildtypinformationen auf der Grundlage von RGB-Signalwerten jedes der mehreren Ultraschallbilder erfasst.

11. Bildanzeigevorrichtung nach Anspruch 8,
wobei die Bildextraktionseinheit (27) ein B-Modus-Bild aus den mehreren Ultraschallbildern auf der Grundlage der Bildtypinformationen extrahiert.

12. Steuerverfahren einer Bildanzeigevorrichtung, wobei das Steuerverfahren umfasst:
Eingeben von mehreren Ultraschallbildern, in denen ein Läsionsteil einer Untersuchungsperson abgebildet ist;
Analysieren der mehreren Ultraschallbilder, um Sondenpositionsinformationen einer Ultraschallsonde und Bildgebungsmerkmalsinformationen in Bezug auf eine Position des Läsionsteils und einen Bildgebungsbereich auf der Grundlage einer Entfernung zwischen Pixeln in jedem der mehreren Ultraschallbilder zu erfassen;
Extrahieren eines Ultraschallbildes, das mit einem von einem Benutzer eingestellten Anzeigelayout konform geht, aus den mehreren Ultraschallbildern unter Bezugnahme auf die erfassten Sondenpositionsinformationen und die erfassten Bildgebungsmerkmalsinformationen, und
Anzeigen des extrahierten Ultraschallbildes auf einem Monitor gemäß dem Anzeigelayout.

## Revendications

1. Appareil d'affichage d'images comprenant :
un moniteur (23);
une unité d'analyse (25, 25A) qui analyse une pluralité d'images échographiques dans lesquelles une partie de lésion (F) d'un sujet est imagée pour acquérir des informations de position d'une sonde ultrasonore (10) à un moment d'imagerie, et des informations de caractéristiques d'imagerie concernant une position de la partie de lésion et une plage d'imagerie sur la base d'une distance entre des pixels dans chacune de la pluralité d'images échographiques ;
une unité d'extraction d'images (27) qui extrait une image échographique conforme à une disposition d'affichage réglée par un utilisateur, à partir de la pluralité d'images échographiques en se référant aux informations de position de sonde et aux informations de caractéristiques d'imagerie acquises par l'unité d'analyse ; et
un contrôleur d'affichage (22) qui affiche l'image échographique extraite par l'unité d'extraction d'images sur le moniteur selon la disposition d'affichage.

2. Appareil d'affichage d'images selon la revendication 1, comprenant en outre :
une sonde ultrasonore (10) ; et
une unité d'acquisition d'images (32) qui acquiert la pluralité d'images échographiques dans lesquelles la partie de lésion du sujet est imagée en utilisant la sonde échographique,
dans lequel l'unité d'analyse analyse la pluralité d'images échographiques acquises par l'unité d'acquisition d'images.

3. Appareil d'affichage d'images selon la revendication 1 ou la revendication 2, comprenant en outre :
un dispositif d'entrée (30) permettant au utilisateur d'effectuer une opération d'entrée ; et
une unité de réglage de disposition d'affichage (28) qui règle la disposition d'affichage sur la base de l'opération d'entrée d'utilisateur via le dispositif d'entrée.

4. Appareil d'affichage d'images selon la revendication 1 ou la revendication 2, comprenant en outre :
une unité d'ajout d'informations (26) qui ajoute les informations de position de sonde et les informations de caractéristiques d'imagerie acquises par l'unité d'analyse à chacune de la pluralité d'images échographiques,
dans lequel l'unité d'extraction d'images extrait l'image échographique en se référant aux informations de position de sonde et aux informations de caractéristiques d'imagerie ajoutées à la pluralité d'images échographiques.

5. Appareil d'affichage d'images selon la revendication 1 ou la revendication 2, dans lequel l'unité d'analyse (25, 25A) acquiert les informations de position de sonde sur la base d'une position de sonde et d'une orientation d'une marque corporelle (51) ajoutée à chacune de la pluralité d'images échographiques.

6. Appareil d'affichage d'images selon la revendication 2,
dans lequel la sonde ultrasonore (10) a un capteur de position (17), et
l'unité d'analyse (25, 25A) acquiert les informations de position de sonde sur la base d'une position de sonde détectée par le capteur de position.

7. Appareil d'affichage d'images selon la revendication 1 ou la revendication 2,
dans lequel l'unité d'analyse (25, 25A) effectue de l'analyse de chacune de la pluralité d'images échographiques pour détecter la partie de lésion et acquérir les informations de caractéristiques d'imagerie concernant la position de la partie de lésion.

8. Appareil d'affichage d'images selon la revendication 1 ou la revendication 2,
dans lequel l'unité d'analyse (25, 25A) acquiert des informations de type d'images indiquant si l'image échographique est une image en mode B ou une autre image, ainsi que les informations de position de sonde et les informations de caractéristiques d'imagerie, et
l'unité d'extraction d'images (27) extrait l'image échographique en se référant aux informations de position de sonde, aux informations de type d'images et aux informations de caractéristiques d'imagerie qui sont acquises par l'unité d'analyse.

9. Appareil d'affichage d'images selon la revendication 8,
dans lequel l'unité d'analyse (25, 25A) acquiert les informations de type d'images sur la base de informations de balise ajoutées à chacune de la pluralité d'images échographiques.

10. Appareil d'affichage d'images selon la revendication 8,
dans lequel l'unité d'analyse (25, 25A) acquiert les informations de type d'images sur la base de valeurs de signal RVB de chacune de la pluralité d'images échographiques.

11. Appareil d'affichage d'images selon la revendication 8,
dans lequel l'unité d'extraction d'images (27) extrait une image en mode B à partir de la pluralité d'images échographiques sur la base des informations de type d'images.

12. Procédé de contrôle d'un appareil d'affichage d'images, le procédé de contrôle comprenant :
entrer une pluralité d'images échographiques dans lesquelles une partie de lésion d'un sujet est imagée ;
analyser la pluralité d'images échographiques pour acquérir des informations de position d'une sonde ultrasonore, et des informations de caractéristiques d'imagerie concernant une position de la partie de lésion et une plage d'imagerie sur la base d'une distance entre des pixels dans chacune de la pluralité d'images échographiques ;
extraire une image échographique conforme à une disposition d'affichage réglée par un utilisateur, à partir de la pluralité d'images échographiques en se référant aux informations de position de sonde acquises et aux informations de caractéristiques d'imagerie acquises ; et
afficher l'image échographique extraite sur un moniteur selon la disposition d'affichage.
